# EUROPEAN PATENT SPECIFICATION

(11) **EP 3 263 080 B1**
(45) Date of publication and mention of the grant of the patent: **19.06.2019**
(21) Application number: 16176747.0
(22) Date of filing: 28.06.2016
(51) Int. Cl.: A61F 13/20, B65G 49/00, B28B 3/00

(54) **TRANSPORT METHOD AND APPARATUS FOR TAMPONS**
TRANSPORTVERFAHREN UND -VORRICHTUNG FÜR TAMPONS
PROCÉDÉ ET APPAREIL DE TRANSPORT POUR TAMPONS

(43) Date of publication of application: 03.01.2018
(73) Proprietor: Ontex BVBA, 9255 Buggenhout (BE)
(72) Inventor: HEEGE, Rudolf, 56759 Kaisersesch (DE); HEEGE, Thomas, 56727 Mayen (DE)
(74) Representative: Larangé, Françoise

(56) References cited:
- EP-A2- 1 304 095
- DE-C1- 19 902 643
- US-A- 3 845 520
- US-A- 3 874 032

## Description

### TECHNICAL FIELD

The invention pertains to the technical field of manufacturing tampons, in particular to an apparatus and method for pressing tampon blanks into compressed tampon pre-forms.

### BACKGROUND

Tampons are well known in the art and are used for feminine hygiene. Also many tampon manufacturing methods and apparatuses have been disclosed in prior art documents. The manufacturing of compressed tampons comprises several subsequent steps, one of which being the pressing step. In this step, so-called tampon blanks, typically coming from a winding station, are pressed into tampon pre-forms, which can then be further processed and packaged. With the multitude of improvements on tampon manufacturing, the applicant has found that the pressing step has become the next major bottleneck for the overall production speed in terms of throughput. Due to the necessary minimum retention time of the tampon blanks in the pressing unit, the speed of a single pressing unit cannot be increased beyond a certain maximum. While this maximum speed is sufficient for the basic production process, the applicant has found that it does not attain the speed of the other steps in the production process, representing a bottleneck.

Seemingly, production speed can be increased by increasing the amount of pressure within the pressing unit, hence reducing the required retention time. This however comes with additional requirements of more durable components for the machine as well as actuators of a higher power. Additionally, this solution could cause unwanted changes in the physical properties of the end product, e.g. with regards to absorbency or comfort.

One way to speed up production is by operating two entire production lines independently, for instance two Ruggli CL2/CL3 production lines. While this is a simple solution, it causes more mechanical breakdowns due to higher component count, it requires more space due to larger footprint, higher investment cost and significantly higher maintenance costs.

Another way to speed up production of a single tampon production line is by pressing multiple tampon blanks at the same time within a production line. The applicant has found that this could indeed speed up the production process. However, tampons thus produced do not follow the same process steps at the same time. Indeed, if two tampon blanks in a single production line are being pressed at the same time, the first tampon blank arrives at the pressing unit before the second tampon blank, and thus has a waiting period before being pressed, compared to the second tampon blank. This waiting period could result in a relaxing of the first tampon blank before being pressed, which can ultimately lead to the first tampon having different characteristics than the second tampon. Vice versa, after pressing, the second tampon pre-form will have a waiting period, as compared to the first tampon pre-form, before being further processed, which again could lead differences in characteristics of the two finished tampons. Although these differences could be small, it is very important in a large-scale production line (whereby tens of thousands to hundreds of thousands tampons are produced per day) to produce each tampon as similarly as possible in order to produce batches of uniform quality.

Prior art methods for speeding up the pressing step in a single production line are mainly directed towards the simultaneous pressing of a plurality of tampon blanks in one or more steps. Document US3874032, for instance, discloses a section of a tampon production line comprising two winding stations, two packing stations and four pressing units in between. The transport device to carry workpieces from the winding station to the presses is an endless conveyor chain with a plurality of cups capable of retaining the workpieces during movement. Similarly, the transport device to carry workpieces from the presses is another endless conveyor chain with a plurality of cups with said aim. According to the disclosed method, tampon blanks are produced by the winding station one by one and are then carried by a first transport device to the presses, where they are simultaneously pressed in pairs of two, to then be carried further by a second transport device. Hence, due to the sequence of single exits from the winding station followed by a pair-wise pressing step, the processing time of two simultaneously-pressed tampons differs by some finite time difference that may result in an undesired difference between them in terms of quality of the end product. Similarly, document US3845520 discloses a tampon manufacturing apparatus wherein a plurality of tampon blanks is carried one by one by a cylindrical workpiece holder of a workpiece carrier to then be pressed simultaneously in two consequent pressing steps. Again, the simultaneous pressing results in finite differences in processing time between simultaneously-pressed tampons, which may result in undesired differences in quality of the end product.

Moreover, the above prior art methods for transferring tampon blanks to and tampon pre-forms from the press are mainly directed towards a linear movement of tampon blanks from a source feed to the press, succeeded by a similar linear path movement of tampon pre-forms from the press to a destination feed. Document US3874032, for instance, discloses such a transfer method, wherein rolls are carried by an endless conveyor chain with a plurality of cups capable of retaining the rolls and tampon pre-forms during linear movement. Similarly, document US3845520 discloses a tampon manufacturing apparatus that includes such a transfer method, wherein pre-shaped workpieces are held by a cylindrical workpiece holder of a workpiece carrier moving in a one-way direction. The linear transfer of the tampon blanks towards the press in these documents lends itself to the type of pressing unit which is used therein. However, other transfer means could be found to optimize the total production process.

Given the above considerations, there remains a need in the art for an improved method and apparatus for production of tampons on a single production line whereby the pressing step is sped up while ensuring that each tampon is produced using the same method steps at the same moment in the production time line of the tampon. Thereby, key process parameters for handling subsequent workpieces should be as constant as possible. The present invention aims to resolve at least some of the problems mentioned above. The invention thereto aims to provide a method and apparatus which can be used in a single production line, the method and apparatus processing tampon blanks into tampon pre-forms in a uniform way leading to constant quality for the end product.

There also remains a need in the art for an improved apparatus for the transfer of tampon blanks from a source feed to a press and for the transfer of tampon pre-forms from the press to a destination feed.

### SUMMARY OF THE INVENTION

The present invention concerns a method for pressing tampon blanks into tampon pre-forms, an apparatus for pressing tampon blanks from a source feed into tampon pre-forms at a destination feed, tampon transfer apparatuses and methods for transferring tampon blanks from a source feed to a plurality of presses and for transferring tampon pre-forms from a plurality of tampon presses to a destination feed.

The applicant has found that an effective solution to speed up the tampon production process on a single production line is to provide a plurality of pressing units which can be fed tampon blanks from a single source feed, e.g. coming from a winding station. Additionally, the plurality of pressing units should feed into a single destination feed, e.g. a packaging station.

This is preferably done in such a way that the key process parameters are impacted as little as possible, and all tampon end products are of equal high quality, without difference amongst them. Key process parameters include the applied pressure and the retention time in individual presses, but also the time it takes to move from one process step to the next, which, in view of the thermal processing involved in tampon production, should be as constant as possible from workpiece to workpiece in order to match the heating and cooling times as required by the process. By using a plurality of pressing units, it becomes possible to do this by operating the pressing units asynchronously.

Thereto, in a first aspect the present invention concerns a method for pressing tampon blanks into tampon pre-forms, comprising the steps of:
(a) subsequently providing at least a first tampon blank and a second tampon blank at a source feed;
(b) transferring said first tampon blank from said source feed to a first tampon press;
(c1) pressing said first tampon blank into a first tampon pre-form;
(c2) transferring said second tampon blank from said source feed to a second tampon press;
(d) pressing said second tampon blank into a second tampon pre-form;
(e) releasing said first tampon pre-form from said first press and transferring said first tampon pre-form from said first press to a destination feed;
(f) releasing said second pre-form from said second press and transferring said second tampon pre-form from said second press to said destination feed
characterized in steps (c1) and (c2) are performed at least partially simultaneously and in that step (d) is performed at least partially simultaneously as step (e), thereby asynchronously pressing the first tampon blank and the second tampon blank.

The method is described in terms of two tampon presses, but can also be applied for more than two tampon presses, e.g. 3, 4, 5, 6 or more, whereby the each of the tampon presses performs the pressing operation asynchronously with each of the other presses.

The invention also provides an apparatus suitable for performing the above method. Thereto, the invention provides an apparatus for pressing tampon blanks from a source feed into tampon pre-forms at a destination feed, comprising a first tampon press, a second tampon press and
- a tampon blank transfer device which is configured to transfer a first tampon blank at the source feed to the first tampon press and subsequently transfer a second tampon blank at the source feed to the second tampon press, whereby said first tampon press is configured to press the first tampon blank into a first tampon pre-form at least partially simultaneously with the transfer of the second tampon blank from the source feed to the second tampon press, and
- a tampon pre-form transfer device which is configured to transfer a first tampon pre-form from the first tampon press to the destination feed and subsequently transfer a second tampon pre-form from the second tampon press to the destination feed, whereby said second tampon press is configured to press the second tampon blank into a second tampon pre-form at least partially simultaneously with the transfer of the first tampon blank from the first tampon press to the destination feed.

The transfer of the tampon blanks from the source feed to the presses and of the tampon pre-forms from the presses to the destination feed is preferably done with a compact and robust method to transfer tampon blanks and tampon pre-forms to and from the presses.

The present invention hereby provides such a compact and robust method of transfer that does not require a separate transfer device per each tampon press, while still allowing for significant speed-up of the production process enabled by the plurality of tampon presses.

Thereto, in a second aspect, the present invention provides a tampon blank transfer device for transferring tampon blanks from a source feed to a number of at least two tampon presses, said device comprising tampon blank holding means, such as tampon blank holes, each of which suitable for receiving and releasing a single tampon blank, preferably during a stop of the tampon blank transfer device, and for retaining the tampon blank during movement of the tampon blank transfer device, wherein the tampon blank transfer device is configured to intermittently and cyclically position each of said tampon blank holding means at said source feed and at least one of said tampon presses,
characterized in that
the tampon blank transfer device comprises a number of tampon blank holding means, said number being two or more and said number being at least equal to said number of tampon presses, and
wherein the device is configured, preferably during a single stop of the tampon blank transfer device, to:
- release a first tampon blank from a first tampon blank holding means of the tampon blank transfer device into one of the tampon presses and receive a second tampon blank from the source feed into a second tampon blank holding means of the tampon blank transfer device; or
- release a first tampon blank from a first tampon blank holding means of the tampon blank transfer device into a first tampon press and release a second tampon blank from a second tampon blank holding means of the tampon blank transfer device into a second tampon press.

In a similar aspect, the invention also provides a tampon pre-form transfer device for transferring tampon pre-forms from a number of at least two tampon presses to a destination feed, said device comprising tampon pre-form holding means, such as tampon pre-form holes, each of which suitable for receiving and releasing a single tampon pre-form, preferably during a stop of the tampon pre-form transfer device, and for retaining the tampon pre-form during movement of the tampon pre-form transfer device,
wherein the tampon pre-form transfer device is configured to intermittently, and cyclically position each of said tampon pre-form holding means at said destination feed and at least one of said tampon presses,
characterized in that
the tampon pre-form transfer device comprises a number of tampon pre-form holding means, said number being two or more and said number being at least equal to said number of tampon presses, and
wherein the device is configured, preferably during a single stop of the tampon pre-form transfer device, to:
- release a first tampon pre-form from a first tampon pre-form holding means of the tampon pre-form transfer device into the destination feed and receive a second tampon pre-form from one of the tampon presses into a second tampon pre-form holding means of the tampon pre-form transfer device; or
- receive a first tampon pre-form from a first tampon press into a first tampon pre-form holding means of the tampon pre-form transfer device and receive a second tampon pre-form from a second tampon press into a second tampon pre-form holding means of the tampon pre-form transfer device.

These transfer devices can be used in a method and/or apparatus for pressing tampon blanks into tampon pre-forms as disclosed in this document. The configuration of the devices to operate intermittently and cyclically, i.e. using a number of stop-start operations in a recurring way, allows to precisely time the production steps, and more in particular to accurately time the pressing step for each tampon blank in a single production line with two, or more, tampon presses.

Moreover, the configuration of the tampon blank transfer device to operate intermittently and cyclically, in combination with the presence of a number of holding means which is at least equal to the number of tampon presses, preferably two, allows a single tampon blank transfer device to provide each tampon press with tampon blanks from a single source feed. Vice versa, a similar configuration of the tampon pre-form transfer device to operate intermittently and cyclically, in combination with the presence of a number of holding means which is at least equal to the number of tampon presses, preferably two, allows a single tampon pre-form transfer device to release the tampon pre-forms from each tampon press to a single destination feed.

Hereby, the applicant notes that a solution to feed a plurality of tampon presses would be the use of a plurality of transfer devices, one per tampon press, working in cooperation with each other. This approach would require a plurality of separate actuators and therefore lead to high investment and operating costs and require more space. Additionally, operating a plurality of transport devices for such an operation increases the complexity of the whole machine as well as increase the statistical risk of a mechanical breakdown.

The applicant has also found that the prior art methods for transferring tampon blanks to and tampon pre-forms from the press are mainly directed towards a linear movement of tampon blanks from a source feed to the press, succeeded by a similar linear path movement of tampon pre-forms from the press to a destination feed. Such a linear movement has turned out not to be optimal for providing two or more presses with tampon blanks from a single source feed, and vice versa, for releasing tampon pre-forms from two or more presses to a single destination feed.

The applicant has found that a pivoting movement of the entirety of tampon blanks to the plurality of presses enables handling more than one tampon blank concurrently with a single compact transfer means. Similarly, the applicant has found a pivoting movement of the entirety of tampon pre-forms from the plurality of presses enables handling more than one tampon pre-form concurrently with a single compact transfer means.

Therefore, in a further aspect, the present invention concerns a method for transferring tampon blanks from a source feed to a number of at least two tampon presses, comprising the steps of:
(i) transferring a first tampon blank from a source feed to a first tampon press by pivoting said first tampon blank around a pivot axis;
(ii) subsequently to step (i), transferring a second tampon blank from said source feed to a second tampon press by pivoting said second tampon blank around said pivot axis;
(iii) pressing said first tampon blank into a first tampon pre-form;
(iv) pressing said second tampon blank into a second tampon pre-form;
(v) releasing said first tampon pre-form from said first press and transferring said first tampon pre-form from said first press to a destination feed;
(vi) releasing said second pre-form from said second press and transferring said second tampon pre-form from said second press to said destination feed.

In a similar aspect, the present invention concerns a method for transferring tampon pre-forms from a number of at least two tampon presses to a destination feed, comprising the steps of:
(I) transferring a first tampon blank from a source feed to a first tampon press and inserting said first tampon blank into said first tampon press;
(II) transferring a second tampon blank from said source feed to a second tampon press and inserting said second tampon blank into said second tampon press;
(III) pressing said first tampon blank into a first tampon pre-form;
(IV) pressing said second tampon blank into a second tampon pre-form;
(V) transferring said first tampon pre-form from said first tampon press to a destination feed by pivoting said first tampon pre-form around a pivot axis;
(VI) subsequently to step (V), transferring said second tampon pre-form from said second tampon press to said destination feed by pivoting said second tampon pre-form around said pivot axis.

In preferred embodiments of the present invention, the tampon blank transfer device and/or the tampon pre-form transfer device are pivoting devices. Hereto, tampon blank transfer device may comprise a pivot axis for transferring the first tampon blank from the source feed to the first tampon press by a pivoting motion around said axis and for subsequently transferring the second tampon blank from the source feed to the second tampon press by a pivoting motion around said pivot axis. Similarly, the tampon pre-form transfer device may comprise a pivot axis for transferring the first tampon pre-form from the first tampon press to the destination feed by a pivoting motion around said axis and for subsequently transferring the second tampon pre-form from the second tampon press to the destination feed by a pivoting motion around said pivot axis.

In embodiments of the system, method and devices of the present invention, the tampon blank is transferred from the source feed to the tampon blank transfer device mechanically or fluido-mechanically. A mechanical transfer can be preferably be achieved by a plunger for pushing a tampon blank from the source feed to or into a holding means of the transfer device. A fluido-mechanical transfer can preferably be achieved by a pressurized gas, preferably pressurized air, whereby the source feed is provided with an ejection system for ejecting a tampon blank from the source feed to or into a holding means of the tampon blank transfer means. Alternatively, or additionally, the tampon blank transfer device can be provided with a fluido-mechanical system which can be arranged to create an underpressure for sucking in a tampon blank from the source feed to or into the holding means. This same fluido-mechanical system, or an additional fluido-mechanical system, can be arranged to also transfer fluido-mechanically the tampon blank from the holding means of the transfer device into one of the presses. Hence, in an embodiment, the tampon blanks are transferred fluido-mechanically from the holding means of the tampon blank transfer device into the presses. Alternatively, the transfer of the tampon blank from the holding means of the tampon blank transfer device into one of the presses can be done mechanically, preferably by a plunger. Similar considerations apply for the transfer of the tampon pre-forms from the presses to the holding means of the tampon preform transfer device, and from the holding means of the tampon preform transfer device to the destination feed: these transfers can be achieved mechanically, e.g. by a plunger for transferring the pressed tampon preform from the press to or into a holding means of the tampon preform transfer device and/or from the holding means of the tampon preform transfer device to the destination feed, or these transfers can be achieved fluidomechanically, e.g. by a fluido-mechanical system provided on the presses, the tampon preform transfer device and/or the destination feed, which works by creating a pressure difference by pressurized gas, preferably pressurized air, and/or by an underpressure.

In the above embodiments, using pressurized air for feeding or emptying the source feed, the holding means of the tampon blank transfer device, the presses, the holding means of the tampon preform transfer device and/or the destination feed, is preferred because the inventors have found that this comes at the benefit of a more stable and less energy demanding process, especially at higher production speeds.

In preferred embodiments of the present invention, the pressing motion of said tampon presses and the pivoting motion of said tampon blank transfer device and/or tampon pre-form transfer device are actuated jointly. This allows better adjustment of the transfer devices to the presses, and furthermore allows to drive the transfer devices and the presses with a single actuator. Hence, in a preferred embodiment, the apparatus of the present invention comprises a central shaft functionally connected to each of the presses, preferably via a lever, and the central shaft functionally connected to the tampon blank transfer device and/or the tampon pre-form transfer device, such that the central shaft is configured to jointly actuate the tampon presses and the tampon blank transfer device and/or the tampon pre-form transfer device.

In preferred embodiments of the present invention, the tampon blanks are pressed radially into tampon pre-forms. Hereto, the presses of the present invention are radial presses. The improvements of the present invention over the prior art methods and apparatuses are particularly pronounced for production lines using radial tampon presses. In particular the use of pivoting transfer devices is preferred for radial tampon presses, as they allow easier and better positioning of the holding means of the devices in front of the press opening for longitudinal insertion of the tampon blank or ejection of the tampon pre-form.

The applicant would like to note that, by altering the Ruggli CL2/CL3 method with the present invention, the production speed can be substantially improved by up to a factor of 2 and even more.

### DESCRIPTION OF FIGURES

**Figure 1** shows an embodiment of a transfer device for two tampon presses with asynchronous operation.
**Figure 2** shows an embodiment comprising transfer devices, rolling station, pressing units and packaging station with asynchronous operation.
**Figure 3** shows an embodiment (front view) of the whole of transfer devices and pressing units.
**Figure 4** shows an embodiment (rear view) of the whole of transfer devices and pressing units.
**Figure 5** shows an embodiment (perspective view) of the whole of transfer devices and pressing units.
**Figure 6** shows an embodiment of a transfer device for three tampon presses with partly synchronous operation.
**Figure 7** shows an embodiment of a transfer device for three tampon presses with asynchronous operation.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The present invention concerns a method for pressing tampon blanks into tampon pre-forms, an apparatus for pressing tampon blanks from a source feed into tampon pre-forms at a destination feed, tampon transfer apparatuses and methods for transferring tampon blanks from a source feed to a plurality of presses and for transferring tampon pre-forms from a plurality of tampon presses to a destination feed, as disclosed above and in the claims.

Further preferred embodiments are presented here below, with reference to the drawings.

In a preferred embodiment of the invention, the key parts of the apparatus are tied together so that they work in unison, the key parts being two tampon presses 1 and 2, two transfer means 3 and 4, and a central shaft 5. Each tampon press is attached to the central shaft by means of a pair of levers 6 and 7, 8 and 9. The press motion of both tampon presses 1 and 2 as well as the pivoting motion of both transfer means 3 and 4 are actuated by the pivoting about the central shaft, which is actuated by an external actuator (not shown in greater detail) that is attached to the central shaft by means of a lever 10.

In a particularly preferred embodiment, the tampon blanks are of the swiss-roll type. These type of tampon blanks can be made by rolling up absorbent material and has an inherent firmness allowing it to be pushed longitudinally into the opening of a pressing apparatus, and the subsequently pressed tampon also has an inherent firmness allowing to be pushed longitudinally out of said pressing apparatus.

The invention is further described by the following non-limiting examples which further illustrate the invention, and are not intended to, nor should they be interpreted to, limit the scope of the invention.

### Example 1: Transfer means to two tampon presses with asynchronous operation

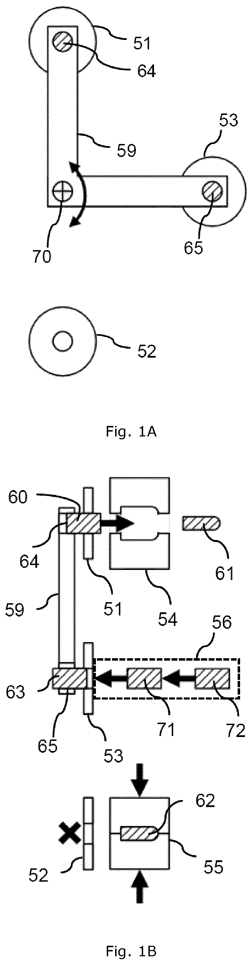

In Fig. 1A-1D, the transfer method and transfer apparatus disclosed in this document are shown in a specific embodiment in which the number of tampon presses is chosen to be equal to two, arranged vertically, hence referred to as top press and bottom press, each of which able to press a tampon blank into a tampon pre product one at a time, i.e. in a sequential manner. Specifically, this example shows an embodiment for transferring tampon blanks from a source feed to a tampon press.

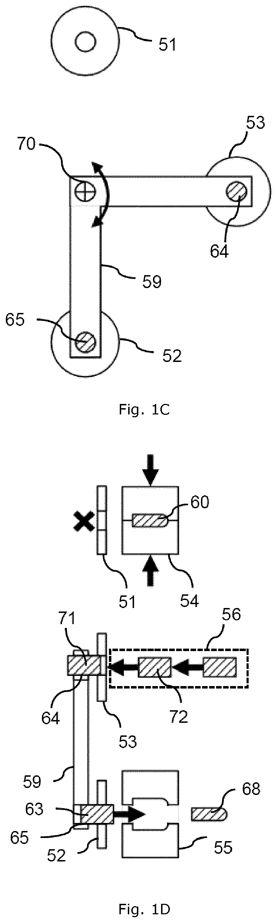

For the transfer of tampon blanks, the transfer process goes through a repeating alternation of stop phases and movement phases, consisting in the repeated execution of a fixed sequence of a first position in a first stop phase, illustrated by Fig. 1A (front view) and Fig. 1B (side view), a first movement phase, a second position in a second stop phase, illustrated by Fig. 1C (front view) and Fig. 1D (side view), and a second movement phase.

Fig. 1A (front view) and Fig. 1B (side view) show said first position of the transfer process, with the arrangement of top press 54 with press mouth 51 and bottom press 55 with press mouth 52, arranged next to a source feed 56 with source feed mouth 53, combined with an L-shaped transfer means 59 with two holes 64 and 65. In said first position, the L-shaped transfer means 59 is in top position and is in a stop phase, characterized by the absence of any pivoting movement of said transfer means. Associated with said first position is the releasing of a tampon blank 60 from hole 64 into the top tampon press 54 which is in an open and thus receiving state. This is preceded by the release of a tampon pre product 61 by the top tampon press 54 during or just before said stop phase. Further associated with said first position is the state of unavailability for receiving tampon blanks of bottom tampon press 55 (whereby the unavailability is depicted with a cross) where the unavailability is due to the pressing of tampon pre product 62 that is ongoing during (and possibly, extending before or after) the stop phase associated with the first position. Further associated with said first position is the receiving of a tampon blank 63 from the source feed 56 into hole 65 of the transfer means 59.

At the end of the stop phase associated with the first position, the transfer process enters said first movement phase (not shown in the figures) whereby the transfer means 59 pivots about its pivot point 70 in clockwise direction changing from top position to bottom position, while retaining a tampon blank 63 in hole 65.

Fig. 1C (front view) and Fig. 1D (side view) show said second position of the transfer process, with the same arrangement of top press 54 with press mouth 51, bottom press 55 with press mouth 52 and source feed 56 with source feed mouth 53, but now with top press 54 in state of unavailability due to ongoing pressing of tampon pre product 60, with bottom press 55 in receiving state, and with transfer means 59 in bottom position and in a stop phase. Associated with said second position is the releasing of a tampon blank 63 from hole 65 into the bottom tampon press 55 which is in a receiving state. Here, this receiving state is preceded by the release of a tampon pre product 68 during or just before said stop phase. Further, said second position implies unavailability of the top tampon press for receiving tampon blanks (whereby the unavailability is marked with a cross) which is due to ongoing pressing. Similar as the first position, said second position is associated with the receiving of a next tampon blank 71 from the source feed 56 into hole 64 of the transfer means 59.

At the end of the stop phase associated with the second position, the transfer process enters said second movement phase (not shown in the figures), whereby the transfer means 59 pivots about its pivot point 70 in counterclockwise direction changing from bottom position to top position, while retaining a tampon blank 71 in hole 64. At the end of the movement phase, the transfer means is back in the first position associated with a stop phase, and a new stop phase associated with the first position starts, wherein the next tampon blank 72 can be received while tampon blank 71 is released, and so on.

### Example 2: Transfer means from two tampon presses with asynchronous operation

This example (not shown in the figures) is identical to Example 1 apart from several aspects. Firstly, the transfer means does not transfer tampon blanks from a source feed to the presses, but instead transfers tampon pre products from the presses to a destination feed. Secondly, the holding means, preferably holes, of the transfer means are not adapted to receiving, releasing and retaining tampon blanks but instead are adapted to perform the same actions on a tampon pre product that has a smaller diameter than a tampon blank. Thirdly, the transfer process is adapted mutatis mutandis', with a transfer from two presses into one feed, instead of a transfer from one feed to two presses.

### Example 3: Combination of two transfer means with asynchronous operation

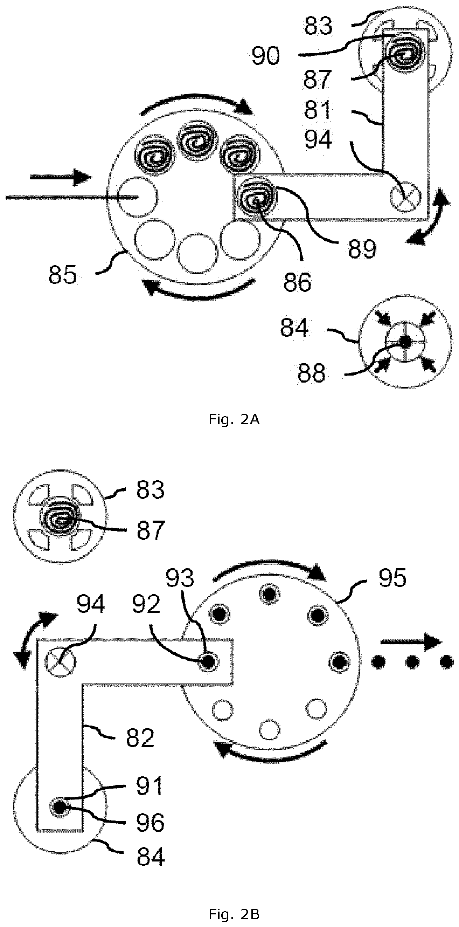

In Fig. 2A and 2B, the transfer methods and transfer apparatuses disclosed in this document are shown in another specific embodiment in which the combination of two transfer means operates on two tampon presses, again arranged vertically, correspondingly referred to as top press and bottom press, each of which able to press a tampon blank into a tampon pre product one at a time.

Fig. 2A shows a front view of the top press 83, the bottom press 84, the first transfer means 81 in top position, and the source feed here embodied as a rolling station 85. Corresponding to the top position of the first transfer means, the top press 83 is in receiving state whereas the bottom press 84 is in unavailable state due to ongoing pressing of tampon blank/pre product 88. The first transfer means 81 receives a tampon blank 86 from rolling station 85, while releasing tampon blank 87 from hole 90 into top tampon press 83. Upon ending of the stop phase, the first transfer means pivots about its pivot point 94 in counterclockwise direction to enter a stop phase in the bottom position, again followed by a moving phase, and so on.

Fig. 2B shows a rear view of the same top press 83, the same bottom press 84, the second transfer means 82 in bottom position, and the destination feed here embodied as a head-forming station 95, i.e. a device wherein the tampon pre-forms are further pressed at a longitudinal end to provide a profiled head, such as a spherical, dome-shaped or mushroom-shaped head. Here, note that the holes 91 and 93 of the second transfer means are somewhat smaller than the holes of the first transfer means to be adapted to the diameter of the tampon pre products, which is smaller than that of the tampon blanks handled by the first transfer means. Also note that the pivot point 94 is shared by both transfer means. Further, corresponding to the bottom position of the second transfer means, the top press 83 is in receiving state whereas the bottom press 84 is in unavailable state due to ongoing pressing of tampon blank/pre product 88. The second transfer means 82 has received a tampon pre product 96 from bottom tampon press 84, while releasing tampon pre product 93 from hole 92 into the head-forming station 95. Upon ending of the stop phase, the second transfer means pivots about its pivot point 94 in counterclockwise direction to enter a stop phase in the top position, again followed by a moving phase, and so on.

While the stop phase of the first and the second transfer means need not be synchronized in the sense of synchronous ending or starting of a stop or movement phase, the total duration of one cycle consisting of a stop phase and a movement phase needs to be the same for both transfer means in order to function properly.

Indeed, by appropriately alternating the phases of the first and second transfer means 81 and 82, the tampon pre product 88 present in the bottom press 84 just after pressing can be released without interference of the first transfer means 81 if the latter is configured to have moved away from the bottom position by that time; likewise, the tampon blank 83 retained by the first transfer means 94 can be released into the tampon press without interference of the second transfer means 82 if the latter is configured to have moved away from the top position by that time. As this alternation allows to work in unison, and the pivot point is shared by both transfer means, the present example illustrates a powerful combination of two methods and apparatuses for transfer disclosed in this document.

### Example 4: Machine comprising two radial tampon presses, two transfer means and a central shaft

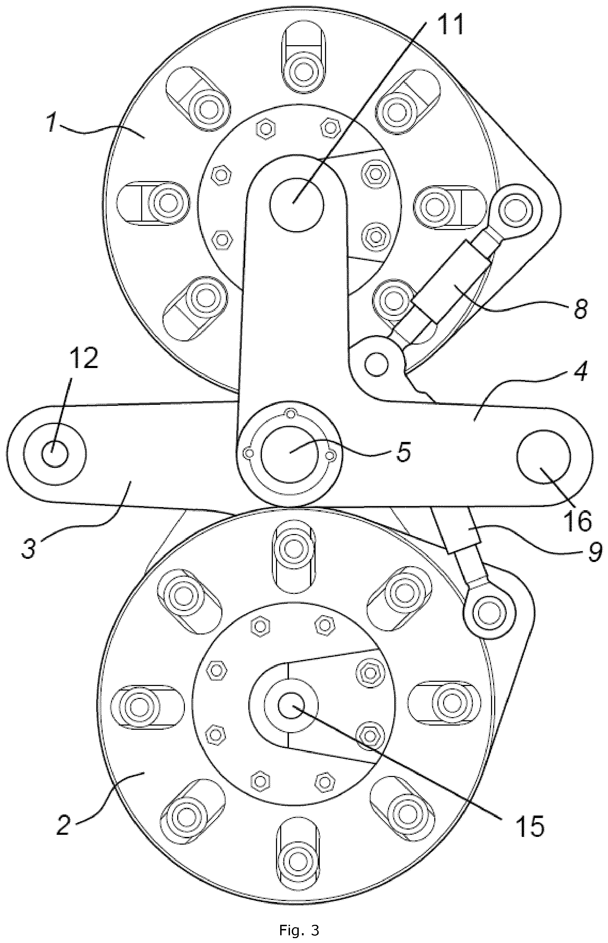

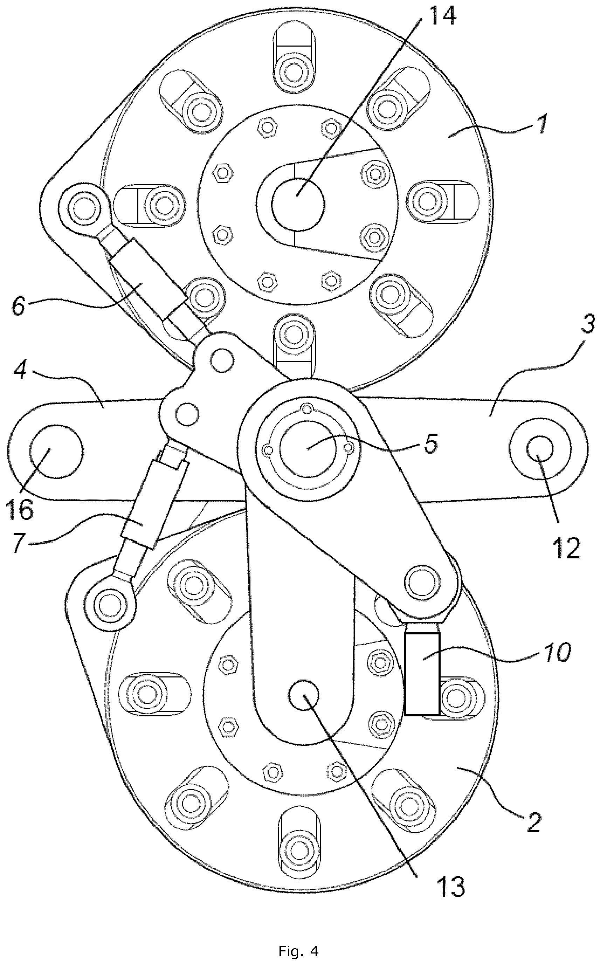

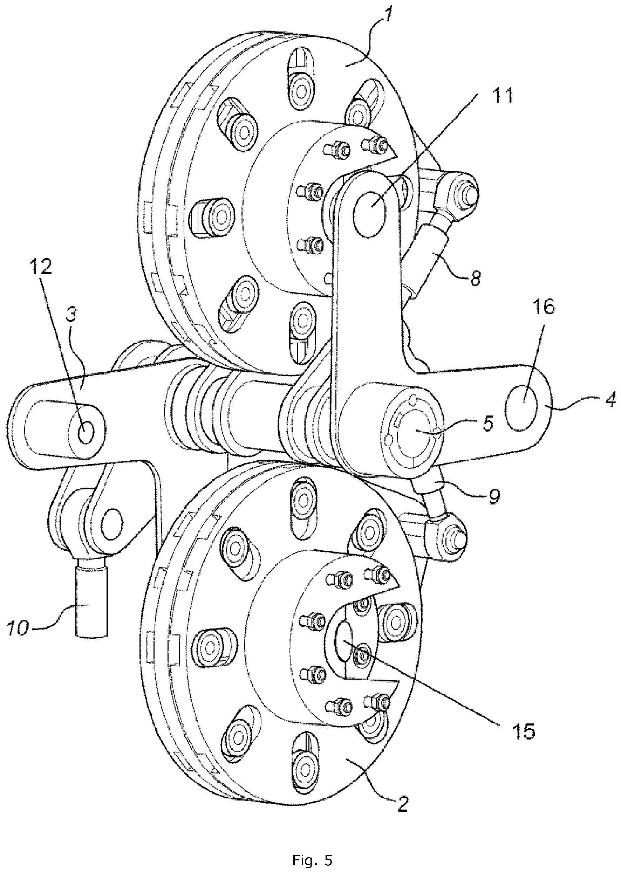

In Fig. 3 (front view), Fig. 4 (rear view) and Fig. 5 (perspective view), the transfer methods and transfer apparatuses disclosed in this document are shown in another specific embodiment, wherein the tampon presses, two in number, are radial pressing units, and said units together with two transfer means are actuated by the pivoting of a central shaft 5. In particular, Fig. 3 (front view) shows the first transfer means 4 intended for receiving a tampon blank from a source feed (not shown) into either of the holes 16 and 11, retaining said tampon blank during pivoting about the central shaft 5 and releasing said tampon blank either from hole 11 into the pressing hole 14 of the top tampon press 1 or from hole 16 into the bottom tampon press 2. Further, Fig. 4 (rear view) shows the second transfer means 3 intended for receiving a tampon pre product from either the pressing hole 14 of the top tampon press 1 into hole 12 or from the pressing hole 15 of the bottom tampon press 2 into hole 13, retaining said tampon pre product during pivoting about the central shaft 5 and releasing said tampon pre product from either of the holes 12 and 13 into the destination feed (not shown). Further, Fig. 5 (perspective view) shows the parts mentioned for Fig. 3 and 4 in a perspective view.

As said, both tampon presses 1 and 2 and both transfer means 3 and 4 are actuated by a single actuator (not shown) that is attached to the central shaft 5 by a lever 10. To this end, the top tampon press is attached to the central shaft with a pair of levers 6 and 8, and likewise for the bottom tampon press that is attached to the central shaft with a pair of levers 7 and 9. Hence, the pressing motion of the rotary pressing units 1 and 2 as well as the pivoting motion of the transfer means 3 and 4 is actuated by the pivoting motion of said shaft 5, which in its turn is actuated by the single actuator (not shown) with the lever 10. The levers 6, 7, 8 and 9 by which the tampon presses are attached to the central shaft 5 are adjustable in length to become longer respectively shorter, effectuating a more intense respectively less intense pressing motion, thereby producing tampon pre products of different diameters, corresponding to different formats for the tampon end products.

### Example 6: Transfer means to three tampon

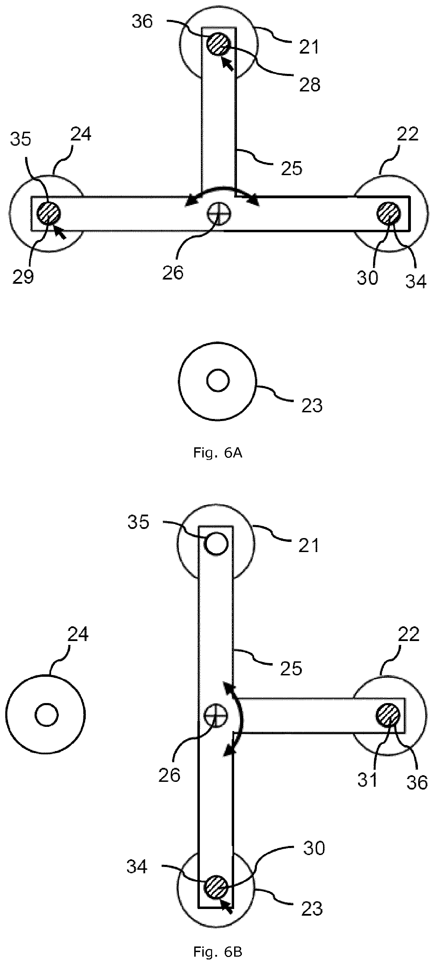

In Fig. 6A-6F, the transfer method and transfer apparatus disclosed in this document are shown in a specific embodiment in which the number of tampon presses is chosen to be equal to three, arranged in a triangle, hence referred to as top press, left press and bottom press, each of which able to press a tampon blank into a tampon pre product one at a time, i.e. in a sequential manner. Specifically, Fig. 6A-6F show an embodiment for transferring tampon blanks from a source feed to a tampon press partly synchronously.

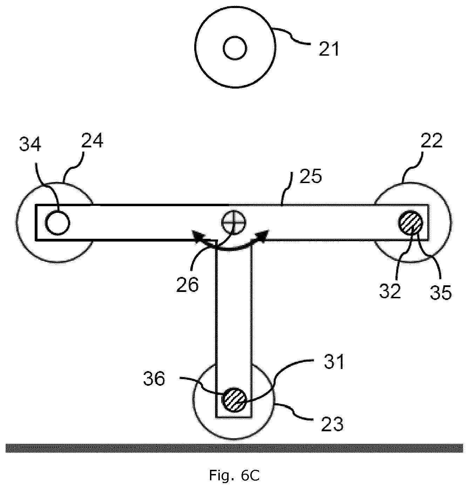

For the transfer of tampon blanks, the transfer process goes through a repeating alternation of stop phases and movement phases, consisting in the repeated execution of a fixed sequence of a first position in first stop phase, illustrated by Fig. 6A (first position), a first movement phase (not shown), a second position in a second stop phase, illustrated by Fig. 6B, a second movement phase, a third position in a third stop phase, illustrated by Fig. 6C, and a third movement phase. Due to the similarities with Example 1, only the front view is shown, as the side view can be derived from it following a similar reasoning as in Example 1.

Fig. 6A shows said first position of the transfer process, with the arrangement of top press 21, bottom press 23 and left press 24 (each press including a press mouth), arranged next to a source feed 22 (including feed mouth), combined with a T-shaped transfer means 25 with three holes 33, 34 and 35. In said first position, the L-shaped transfer means 25 is in top position and is in a stop phase, characterized by the absence of any pivoting movement of said transfer means. Associated with said first position is the releasing of a tampon blank 28 from hole 35 into the top tampon press 21 which is in an open and thus receiving state (depicted with an arrow), as well as the releasing of a tampon blank 29 from hole 29 into the left tampon press 24 which is also in a receiving state (depicted with an arrow). Further associated with said first position is the state of unavailability for receiving tampon blanks of bottom tampon press 23 where the unavailability is due to the pressing of a tampon pre product (not shown) that is ongoing during (and possibly, extending before or after) the stop phase associated with the first position. Further associated with said first position is the receiving of a tampon blank 30 from the source feed 22 into hole 33 of the transfer means 25.

At the end of the stop phase associated with the first position, the transfer process enters said first movement phase (not shown in the figures) whereby the transfer means 25 pivots about its pivot point 26 in clockwise direction changing from top position to left position, while retaining a tampon blank 30 in hole 33.

Fig. 6B shows said second position of the transfer process, in which the L-shaped transfer means 25 is in left position and is in a stop phase. Associated with said second position is the releasing of a tampon blank 30 from hole 33 into the bottom tampon press 23 which is in a receiving state. Further associated with said second position is the state of unavailability for receiving tampon blanks of left tampon press 24 as well as top tampon press 21 where the unavailability is due to the pressing of a tampon pre product (not shown). Further associated with said second position is the receiving of a tampon blank 31 from the source feed 22 into hole 35 of the transfer means 25.

At the end of the stop phase associated with the second position, the transfer process enters said second movement phase (not shown in the figures) whereby the transfer means 25 pivots about its pivot point 26 in clockwise direction changing from left position to bottom position, while retaining a tampon blank 31 in hole 35.

Fig. 6B shows said third position of the transfer process, in which the L-shaped transfer means 25 is in bottom position and is in a stop phase. In said second position, no tampon blank is released and all three tampon presses 21, 23 and 24 are unavailable where the unavailability is due to the pressing of a tampon pre product (not shown). Further associated with said third position is the receiving of a tampon blank 32 from the source feed 22 into hole 34 of the transfer means 25.

At the end of the stop phase associated with the third position, the transfer process enters said second third phase (not shown in the figures), whereby the transfer means 25 pivots about its pivot point 26 changing from bottom position to top position, either in clockwise or in counterclockwise direction, while retaining a tampon blank 31 in hole 35 as well as retaining a tampon blank 30 in hole 33. At the end of the movement phase, the transfer means is back in the first position associated with a stop phase, and a new stop phase associated with the first position starts, wherein the next tampon blank (not shown) can be received while tampon blank 30 and 31 are released, and so on.

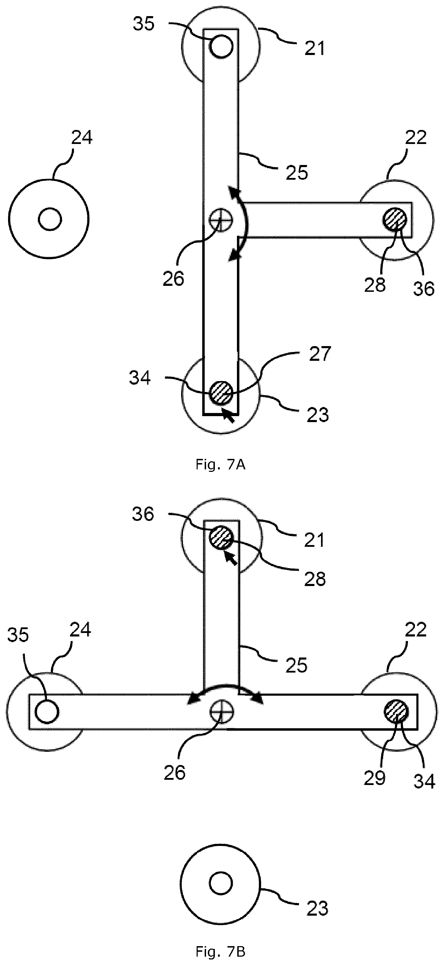

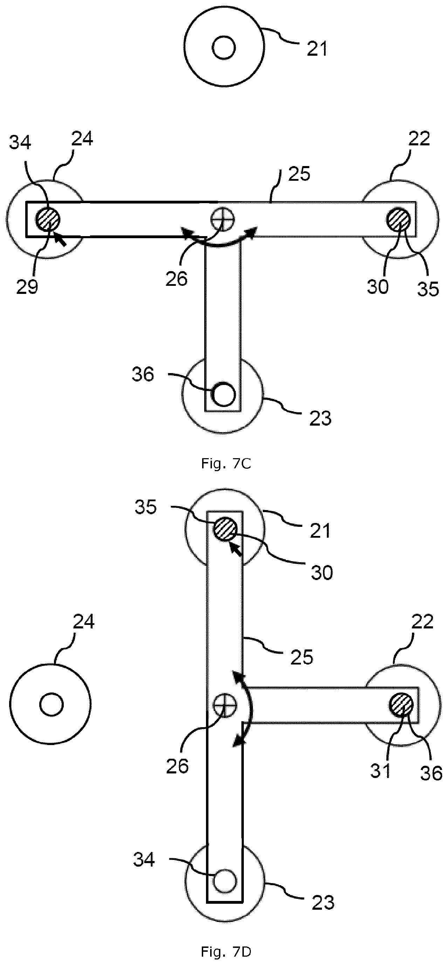

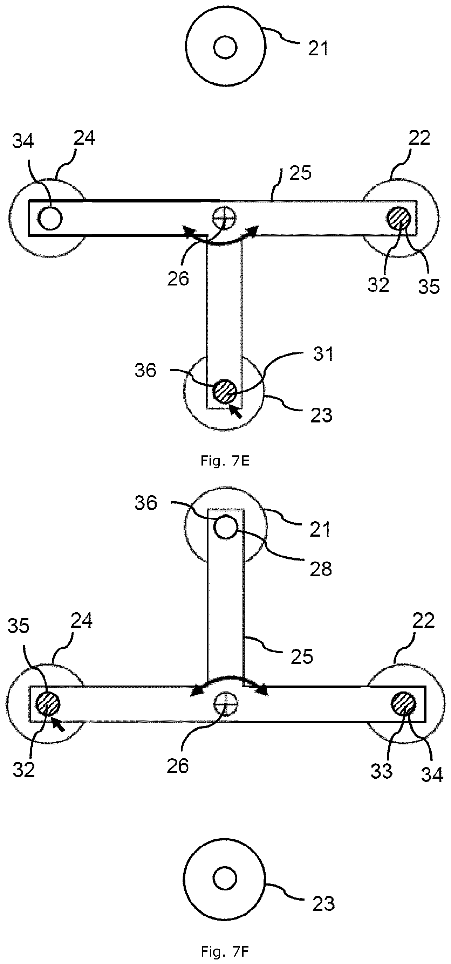

In an alternative embodiment illustrated in Fig. 7A-7F, the same apparatus is configured to perform the transfer asynchronously, consisting in the repeating alternation of six stop phases each followed by a movement phase. The six respective stop phases and corresponding six positions are illustrated in Fig. 7A to 7F, respectively. The figures are self-explaining.

## Claims

1. Method for pressing tampon blanks into tampon pre-forms, comprising the steps of:
(a) subsequently providing at least a first tampon blank and a second tampon blank at a source feed;
(b) transferring said first tampon blank from said source feed to a first tampon press;
(c1) pressing said first tampon blank into a first tampon pre-form;
(c2) transferring said second tampon blank from said source feed to a second tampon press;
(d) pressing said second tampon blank into a second tampon pre-form;
(e) releasing said first tampon pre-form from said first press and transferring said first tampon pre-form from said first press to a destination feed;
(f) releasing said second pre-form from said second press and transferring said second tampon pre-form from said second press to said destination feed
**characterized in that** steps (c1) and (c2) are performed at least partially simultaneously and **in that** step (d) is performed at least partially simultaneously as step (e), thereby asynchronously pressing the first tampon blank and the second tampon blank.

2. Method according to claim 1, wherein the transfer of the first tampon blank to the first tampon press in step (b) and the transfer of the second tampon blank to the second tampon press in step (c) is performed by a pivoting tampon blank transfer device, and/or wherein the transfer of said first tampon pre-form from said first press to a destination feed in step (e) and the transfer of said second tampon pre-form from said second press to said destination feed in step (f) is performed by a pivoting tampon pre-form transfer device.

3. Method according to claim 2, wherein the pressing motion of said tampon presses and the pivoting motion of said tampon blank transfer device and/or tampon pre-form transfer device are actuated jointly.

4. Method according to any of claims 1 to 3, wherein said tampon blanks are pressed radially into tampon pre-forms.

5. Apparatus for pressing tampon blanks from a source feed into tampon pre-forms at a destination feed, comprising a first tampon press, a second tampon press and
- a tampon blank transfer device which is configured to transfer a first tampon blank at the source feed to the first tampon press and subsequently transfer a second tampon blank at the source feed to the second tampon press, **characterized in that** said first tampon press is configured to press the first tampon blank into a first tampon pre-form at least partially simultaneously with the transfer of the second tampon blank from the source feed to the second tampon press, and
- a tampon pre-form transfer device which is configured to transfer a first tampon pre-form from the first tampon press to the destination feed and subsequently transfer a second tampon pre-form from the second tampon press to the destination feed, **characterized in that** said second tampon press is configured to press the second tampon blank into a second tampon pre-form at least partially simultaneously with the transfer of the first tampon blank from the first tampon press to the destination feed.

6. Apparatus according to claim 5, wherein said tampon blank transfer device comprises at least two tampon blank holding means, such as tampon blank holes, each of which suitable for receiving and releasing a single tampon blank during a stop of the tampon blank transfer device and for retaining the tampon blank during movement of the tampon blank transfer device and wherein said tampon pre-form transfer device comprises at least two tampon pre-form holding means, such a tampon pre-form holes, each of which suitable for receiving and releasing a single tampon pre-form during a stop of the tampon pre-form transfer device and for retaining the tampon pre-form during movement of the tampon pre-form transfer device.

7. Apparatus according to claim 6, wherein the tampon blank transfer device is configured to intermittently and cyclically position each of said tampon blank holding means in front of said source feed such that a tampon blank can be inserted longitudinally into one of said tampon blank holding means during a stop of said tampon blank transfer device, and/or wherein the tampon pre-form transfer device is configured to intermittently and cyclically position each of said tampon pre-form holding means in front of said destination feed such that a tampon pre-form can be released longitudinally from one of said tampon pre-form holding means during a stop of said tampon pre-form transfer device.

8. Apparatus according to any of claims 5 to 7, wherein said tampon blank transfer device comprises a pivot axis for transferring the first tampon blank from the source feed to the first tampon press by a pivoting motion around said axis and for subsequently transferring the second tampon blank from the source feed to the second tampon press by a pivoting motion around said pivot axis.

9. Apparatus according to any of claims 5 to 8, wherein said tampon pre-form transfer device comprises a pivot axis for transferring the first tampon pre-form from the first tampon press to the destination feed by a pivoting motion around said axis and for subsequently transferring the second tampon pre-form from the second tampon press to the destination feed by a pivoting motion around said pivot axis.

10. Apparatus according to any of claims 5 to 9, wherein said tampon presses are radial tampon presses.

11. Apparatus according to any of claims 5 to 10, comprising a central shaft functionally connected to each of said presses, preferably via a lever, and the central shaft functionally connected to the tampon blank transfer device and/or the tampon pre-form transfer device, such that the central shaft is configured to jointly actuate the tampon presses and the tampon blank transfer device and/or the tampon pre-form transfer device.

12. Tampon blank transfer device for transferring tampon blanks from a source feed to a number of at least two tampon presses, said device comprising tampon blank holding means, such as tampon blank holes, each of which suitable for receiving and releasing a single tampon blank during a stop of the tampon blank transfer device and for retaining the tampon blank during movement of the tampon blank transfer device,
wherein the tampon blank transfer device is configured to intermittently and cyclically position each of said tampon blank holding means at said source feed and at least one of said tampon presses,
**characterized in that**
the tampon blank transfer device comprises a number of tampon blank holding means, said number being two or more and said number being at least equal to said number of tampon presses, and
wherein the device is configured, during a single stop of the tampon blank transfer device, to:
- release a first tampon blank from a first tampon blank holding means of the tampon blank transfer device into one of the tampon presses and receive a second tampon blank from the source feed into a second tampon blank holding means of the tampon blank transfer device; or
- release a first tampon blank from a first tampon blank holding means of the tampon blank transfer device into a first tampon press and release a second tampon blank from a second tampon blank holding means of the tampon blank transfer device into a second tampon press.

13. Tampon pre-form transfer device for transferring tampon pre-forms from a number of at least two tampon presses to a destination feed, said device comprising tampon pre-form holding means, such as tampon pre-form holes, each of which suitable for receiving and releasing a single tampon pre-form during a stop of the tampon pre-form transfer device and for retaining the tampon pre-form during movement of the tampon pre-form transfer device,
wherein the tampon pre-form transfer device is configured to intermittently, and cyclically position each of said tampon pre-form holding means at said destination feed and at least one of said tampon presses,
**characterized in that**
the tampon pre-form transfer device comprises a number of tampon pre-form holding means, said number being two or more and said number being at least equal to said number of tampon presses, and
wherein the device is configured, during a single stop of the tampon pre-form transfer device, to:
- release a first tampon pre-form from a first tampon pre-form holding means of the tampon pre-form transfer device into the destination feed and receive a second tampon pre-form from one of the tampon presses into a second tampon pre-form holding means of the tampon pre-form transfer device; or
- receive a first tampon pre-form from a first tampon press into a first tampon pre-form holding means of the tampon pre-form transfer device and receive a second tampon pre-form from a second tampon press into a second tampon pre-form holding means of the tampon pre-form transfer device.

14. Method for transferring tampon blanks from a source feed to a number of at least two tampon presses, comprising the steps of:
(i) transferring a first tampon blank from a source feed to a first tampon press by pivoting said first tampon blank around a pivot axis;
(ii) transferring a second tampon blank from said source feed to a second tampon press **characterized in that** said transfer is performed subsequently to step (i) by pivoting said second tampon blank around said pivot axis;
(iii) pressing said first tampon blank into a first tampon pre-form;
(iv) pressing said second tampon blank into a second tampon pre-form;
(v) releasing said first tampon pre-form from said first press and transferring said first tampon pre-form from said first press to a destination feed;
(vi) releasing said second pre-form from said second press and transferring said second tampon pre-form from said second press to said destination feed.

15. Method for transferring tampon pre-forms from a number of at least two tampon presses to a destination feed, comprising the steps of:
(I) transferring a first tampon blank from a source feed to a first tampon press and inserting said first tampon blank into said first tampon press;
(II) transferring a second tampon blank from said source feed to a second tampon press and inserting said second tampon blank into said second tampon press;
(III) pressing said first tampon blank into a first tampon pre-form;
(IV) pressing said second tampon blank into a second tampon pre-form;
(V) transferring said first tampon pre-form from said first tampon press to a destination feed by pivoting said first tampon pre-form around a pivot axis;
(VI) transferring said second tampon pre-form from said second tampon press to said destination feed, **characterized in that** said transfer is performed subsequently to step (V) by pivoting said second tampon pre-form around said pivot axis.

## Patentansprüche

1. Verfahren zum Verpressen von Tamponrohlingen zu Tamponvorförmlingen, umfassend die Schritte:
(a) anschließendes Vorsehen mindestens eines ersten Tamponrohlings und eines zweiten Tamponrohlings in einer Zufuhrquelle;
(b) Übergeben des ersten Tamponrohlings von der Zufuhrquelle an eine erste Tampondruckmaschine;
(c1) Verpressen des ersten Tamponrohlings zu einem ersten Tamponvorförmling;
(c2) Übergeben des zweiten Tamponrohlings von der Zufuhrquelle an eine zweite Tampondruckmaschine;
(d) Verpressen des zweiten Tamponrohlings zu einem zweiten Tamponvorförmling;
(e) Freigeben des ersten Tamponvorförmlings aus der ersten Druckmaschine und Übergeben des ersten Tamponvorförmlings von der ersten Druckmaschine an einen Zieltransport;
(f) Freigeben des zweiten Tamponvorförmlings aus der zweiten Druckmaschine und Übergeben des zweiten Tamponvorförmlings von der zweiten Druckmaschine an einen Zieltransport;
**dadurch gekennzeichnet, dass** die Schritte (c1) und (c2) mindestens teilweise gleichzeitig ausgeführt werden, und dass der Schritt (d) mindestens teilweise gleichzeitig mit dem Schritt (e) ausgeführt wird, wodurch der erste Tamponrohlung und der zweite Tamponrohling asynchron gepresst werden.

2. Verfahren nach Anspruch 1, wobei die Übergabe des ersten Tamponrohlings an die erste Tampondruckmaschine im Schritt (b) und die Übergabe des zweiten Tamponrohlings an die zweite Tampondruckmaschine im Schritt (c) durch eine schwenkende Tamponrohlingstransfervorrichtung ausgeführt werden, und/oder wobei die Übergabe des ersten Tamponvorförmlings von der ersten Druckmaschine an einen Zieltransport im Schritt (e) und die Übergabe des zweiten Tamponvorförmlings von der zweiten Druckmaschine an den Zieltransport im Schritt (f) durch eine schwenkdende Tamponvorförmlingstransfervorrichtung ausgeführt werden.

3. Verfahren nach Anspruch 2, wobei die Pressbewegung der Tampondruckmaschinen und die Schwenkung der Tamponrohlingstransfervorrichtung und/oder der Tamponvorförmlingstransfervorrichtung gemeinsam betätigt werden.

4. Verfahren nach einem der Ansprüche 1 - 3, wobei die Tamponrohlinge radial zu Tamponvorförmlingen verpresst werden.

5. Vorrichtung zum Verpressen von Tamponrohlingen aus einer Zufuhrquelle zu Tamponvorförmlingen an einem Zieltransport, umfassend eine erste Tampondruckmaschine, eine zweite Tampondruckmaschine und
- eine Tamponrohlingstransfervorrichtung, die zur Ausführung folgender Funktionen konfiguriert ist: Übergeben eines ersten Tamponrohlings an der Zufuhrquelle an die erste Tampondruckmaschine und anschließendes Übergeben eines zweiten Tamponrohlings an der Zufuhrquelle an die zweite Tampondruckmascine,
**dadurch gekennzeichnet, dass** die erste Tampondruckmaschine derart konfiguriert ist, dass sie mindestens z.T. gleichzeitig mit der Übergabe des zweiten Tamponrohlings von der Zufuhrquelle an die zweite Tampondruckmaschine den ersten Tamponrohling zu einem ersten Tamponvorförmling verpresst; und
- eine Tamponvorförmlingstransfervorrichtung, die zur Ausführung folgender Funktionen konfiguriert ist: Übergeben eines ersten Tamponvorförmlings von der ersten Tampondruckmaschine an den Zieltransport und anschließendes Übergeben eines zweiten Tamponvorförmlings von der zweiten Tampondruckmaschine an den Zieltransport,
**dadurch gekennzeichnet, dass** die zweite Tampondruckmaschine derart konfiguriert ist, dass sie mindestens z.T. gleichzeitig mit der Übergabe des ersten Tamponrohlings von der ersten Tampondruckmaschine an den Zieltransport den zweiten Tamponrohling zu einem zweiten Tamponvorförmling verpresst.

6. Vorrichtung nach Anspruch 5, wobei die Rohlingstransfervorrichtung mindestens zwei Haltemittel für Tamponrohlinge, z.B. Löcher, umfasst, die jeweils dazu geeignet sind, bei einem Stopp der Rohlingstransfervorrichtung einen einzelnen Tamponrohling aufzunehmen und freizugeben und bei Bewegung der Rohlingstransfervorrichtung den Tamponrohling festzuhalten, und wobei die Vorförmlingsstransfervorrichtung mindestens zwei Haltemittel für Tamponvorförmlinge, z.B. Löcher, umfasst, die jeweils dazu geeignet sind, bei einem Stopp der Vorförmlingstransfervorrichtung einen einzelnen Tamponvorförmling aufzunehmen und freizugeben und bei Bewegung der Vorförmlingstransfervorrichtung den Tamponvorförmling festzuhalten.

7. Vorrichtung nach Anspruch 6, wobei die Rohlingstransfervorrichtung derart konfiguriert ist, dass sie jedes der Haltemittel intermittierend und zyklisch vor der Zufuhrquelle positioniert, sodass ein Tamponrohling bei einem Stopp der Rohlingstransfervorrichtung in Längsrichtung in eines der Haltemittel eingeführt werden kann, und/oder wobei die Vorförmlingstransfervorrichtung derart konfiguriert ist, dass sie jedes der Haltemittel intermittierend und zyklisch vor dem Zieltransport positioniert, sodass ein Tamponvorförmling bei einem Stopp der Vorförmlingstransfervorrichtung in Längsrichtung aus einem der Haltemittel freigegeben werden kann.

8. Vorrichtung nach einem der Ansprüche 5 - 7, wobei die Rohlingstransfervorrichtung eine Schwenkachse umfasst, um den ersten Tamponrohling mittels einer Schwenkung um die Achse von der Zufuhrquelle an die erste Tampondruckmaschine zu übergeben und anschließend den zweiten Tamponrohling mittels einer Schwenkung um die Schwenkachse von der Zufuhrquelle an die zweite Tampondruckmaschine zu übergeben.

9. Vorrichtung nach einem der Ansprüche 5 - 8, wobei die Vorförmlingstransfervorrichtung eine Schwenkachse umfasst, um den ersten Tamponvorförmling mittels einer Schwenkung um die Achse von der ersten Tampondruckmaschine an den Zieltransport zu übergeben und anschließend den zweiten Tamponvorförmling mittels einer Schwenkung um die Schwenkachse von der zweiten Tampondruckmaschine an den Zieltransport zu übergeben.

10. Vorrichtung nach einem der Ansprüche 5 - 9, wobei die Tampondruckmaschinen radiale Tampondruckmaschinen sind.

11. Vorrichtung nach einem der Ansprüche 5 - 10, umfassend eine Zentralwelle, die mit jeder der Druckmaschinen, vorzugsweise über einen Hebel, in Wirkverbindung steht, wobei die Zentralwelle mit der Rohlings- und/oder Vorförmlingstransfervorrichtung in Wirkverbindung steht, sodass die Zentralwelle derart konfiguriert ist, dass sie die Tampondruckmaschinen und die Rohlings- und/oder Vorförmlingstransvervorrichtung gemeinsam betätigt.

12. Tamponrohlingstransfervorrichtung zum Übergeben von Tamponrohlingen von einer Zufuhrquelle an mindestens zwei Tampondruckmaschinen, wobei die Vorrichtung Haltemittel für Tamponrohlinge, z.B. Löcher, umfasst, die jeweils dazu geeignet sind, bei einem Stopp der Rohlingstransfervorrichtung einen einzelnen Tamponrohling aufzunehmen und freizugeben und bei Bewegung der Rohlingstransfervorrichtung den Tamponrohling festzuhalten,
wobei die Rohlingstransfervorrichtung derart konfiguriert ist, dass sie jedes der Haltemittel intermittierend und zyklisch an der Zufuhrquelle und mindestens einer der Tampondruckmaschinen positioniert,
**dadurch gekennzeichnet, dass**
die Rohlingstransfervorrichtung Haltemittel umfasst, deren Anzahl mindestens zwei beträgt und mindestens gleich der Anzahl Tampondruckmaschinen ist, und
wobei die Vorrichtung derart konfiguriert ist, dass sie bei einem einzelnen Stopp der Rohlingstransfervorrichtung:
- einen ersten Tamponrohling von einem ersten Haltemittel der Rohlingstransfervorrichtung an eine der Tampondruckmaschinen abgibt und einen zweiten Tamponrohling von der Zufuhrquelle in ein zweites Haltemittel der Rohlingstransfervorrichtung aufnimmt, oder
- einen ersten Tamponrohling von einem ersten Haltemittel der Rohlingstransfervorrichtung an eine erste Tampondruckmaschine abgibt und einen zweiten Tamponrohling von einem zweiten Haltemittel der Rohlingstransfervorrichtung an eine zweite Tampondruckmaschine abgibt.

13. Tamponvorförmlingstransfervorrichtung zum Übergeben von Tamponvorförmlingen von mindestens zwei Tampondruckmaschinen an einen Zieltransport, die Vorrichtung Haltemittel für Tamponrohlinge, z.B. Löcher, umfasst, die jeweils dazu geeignet sind, bei einem Stopp der Rohlingstransfervorrichtung einen einzelnen Tamponrohling aufzunehmen und freizugeben und bei Bewegung der Rohlingstransfervorrichtung den Tamponrohling festzuhalten,
wobei die Rohlingstransfervorrichtung derart konfiguriert ist, dass sie jedes der Haltemittel intermittierend und zyklisch an der Zufuhrquelle und mindestens einer der Tampondruckmaschinen positioniert,
**dadurch gekennzeichnet, dass**
die Vorförmlingstransfervorrichtung Haltemittel umfasst, deren Anzahl mindestens zwei beträgt und mindestens gleich der Anzahl Tampondruckmaschinen ist, und
wobei die Vorrichtung derart konfiguriert ist, dass sie bei einem einzelnen Stopp der Vorförmlingstransfervorrichtung:
- einen ersten Tamponvorförmling von einem ersten Haltemittel der Vorförmlingstransfervorrichtung an den Zieltransport abgibt und einen zweiten Tamponvorförmling von einer der Tampondruckmaschinen in ein zweites Haltemittel der Vorförmlingstransfervorrichtung aufnimmt, oder
- einen ersten Tamponvorförmling von einer ersten Tampondruckmaschine in ein erstes Haltemittel der Vorförmlingstransfervorrichtung aufnimmt und einen zweiten Tamponvorförmling von der zweiten Tampondruckmaschine in ein zweites Haltemittel der Vorförmlingstransfervorrichtung aufnimmt.

14. Verfahren zum Übergeben von Tamponrohlingen von einer Zufuhrquelle an mindestens zwei Tampondruckmaschinen, umfassend die Schritte:
(i) Übergeben eines ersten Tamponrohlings von einer Zufuhrquelle an eine erste Tampondruckmaschine durch Schwenken des Tamponrohlings um eine Schwenkachse;
(ii) Übergeben eines zweiten Tamponrohlings von der Zufuhrquelle an eine zweite Tampondruckmaschine, **dadurch gekennzeichnet, dass** die Übergabe im Anschluss an den Schritt (i) durch Schwenken des zweiten Tamponrohlings um die Schwenkachse ausgeführt wird;
(iii) Verpressen des ersten Tamponrohlings zu einem ersten Tamponvorförmling;
(iv) Verpressen des zweiten Tamponrohlings zu einem zweiten Tamponvorförmling;
(v) Freigeben des ersten Tamponvorförmlings aus der ersten Druckmaschine und Übergeben des ersten Tamponvorförmlings von der ersten Druckmaschine an einen Zieltransport;
(vi) Freigeben des zweiten Tamponvorförmlings aus der zweiten Druckmaschine und Übergeben des zweiten Tamponvorförmlings von der zweiten Druckmaschine an einen Zieltransport.

15. Verfahren zum Übergeben von Tamponvorförmlingen von mindestens zwei Tampondruckmaschinen an einen Zieltransport, umfassend die Schritte:
(I) Übergeben eines ersten Tamponrohlings von einer Zufuhrquelle an eine erste Tampondruckmaschine und Einführen des ersten Tamponrohlings in die erste Tampondruckmaschine;
(II) Übergeben eines zweiten Tamponrohlings von einer Zufuhrquelle an eine zweite Tampondruckmaschine und Einführen des zweiten Tamponrohlings in die zweite Tampondruckmaschine;
(III) Verpressen des ersten Tamponrohlings zu einem ersten Tamponvorförmling;
(IV) Verpressen des zweiten Tamponrohlings zu einem zweiten Tamponvorförmling;
(V) Übergeben des ersten Tamponvorförmlings von der ersten Tampondruckmaschine an einen Zieltransport durch Schwenken des ersten Tamponvorförmlings um eine Schwenkachse;
(VI) Übergeben des zweiten Tamponvorförmlings von der zweiten Tampondruckmaschine an den Zieltransport, **dadurch gekennzeichnet, dass** die Übergabe im Anschluss an den Schritt (V) durch Schwenken des zweiten Tamponvorförmlings um die Schwenkachse ausgeführt wird.

## Revendications

1. Procédé pour comprimer des ébauches de tampon dans des préformes de tampon, comprenant les étapes consistant à :
(a) prévoir successivement au moins une première ébauche de tampon et une seconde ébauche de tampon au niveau d'une alimentation de source ;
(b) transférer ladite première ébauche de tampon de ladite alimentation de source à une première presse de tampon ;
(c1) comprimer ladite première ébauche de tampon dans une première préforme de tampon ;
(c2) transférer ladite seconde ébauche de tampon de ladite alimentation de source à une seconde presse de tampon ;
(d) comprimer ladite seconde ébauche de tampon dans une seconde préforme de tampon ;
(e) libérer ladite première préforme de tampon de ladite première presse et transférer ladite première préforme de tampon de ladite première presse à une alimentation de destination ;
(f) libérer ladite seconde préforme de ladite seconde presse et transférer ladite seconde préforme de tampon de ladite seconde presse à ladite alimentation de destination,
**caractérisé en ce que** les étapes (c1) et (c2) sont réalisées au moins partiellement simultanément et **en ce que** l'étape (d) est réalisée au moins partiellement en même temps que l'étape (e), comprimant ainsi de manière asynchrone la première ébauche de tampon et ladite seconde ébauche de tampon.

2. Procédé selon la revendication 1, dans lequel le transfert de la première ébauche de tampon à la première presse de tampon à l'étape (b) et le transfert de la seconde ébauche de tampon à la seconde presse de tampon à l'étape (c) sont réalisés par un dispositif de transfert d'ébauche de tampon pivotant, et/ou dans lequel le transfert de ladite première préforme de tampon de ladite presse à une alimentation de destination à l'étape (e) et le transfert de ladite seconde préforme de tampon de ladite seconde presse à ladite alimentation de destination à l'étape (f) sont réalisés par un dispositif de transfert de préforme de tampon pivotant.

3. Procédé selon la revendication 2, dans lequel le mouvement de pression desdites presses de tampon et le mouvement pivotant dudit dispositif de transfert d'ébauche de tampon et/ou du dispositif de transfert de préforme de tampon sont actionnés conjointement.

4. Procédé selon l'une quelconque des revendications 1 à 3, dans lequel lesdites ébauches de tampon sont comprimées radialement dans des préformes de tampon.

5. Appareil pour comprimer des ébauches de tampon d'une alimentation de source dans des préformes de tampon à une alimentation de destination, comprenant une première presse de tampon, une seconde presse de tampon, et
un dispositif de transfert d'ébauche de tampon qui est configuré pour transférer une première ébauche de tampon au niveau de l'alimentation de source à une presse de tampon et transférer ensuite une seconde ébauche de tampon au niveau de l'alimentation de source à la seconde presse de tampon,
**caractérisé en ce que** ladite première presse de tampon est configurée pour comprimer la première ébauche de tampon dans une première préforme de tampon au moins simultanément avec le transfert de la seconde ébauche de tampon de l'alimentation de source à la seconde presse de tampon, et
un dispositif de transfert de préforme de tampon qui est configuré pour transférer une première préforme de tampon de la première presse de tampon à l'alimentation de destination et transférer ensuite une seconde préforme de tampon de la seconde presse de tampon à l'alimentation de destination, **caractérisé en ce que** ladite seconde presse de tampon est configurée pour comprimer la seconde ébauche de tampon dans une seconde préforme de tampon au moins partiellement simultanément avec le transfert de la première ébauche de tampon de la première presse de tampon à l'alimentation de destination.

6. Appareil selon la revendication 5, dans lequel ledit dispositif de transfert d'ébauche de tampon comprend au moins deux moyens de support d'ébauche de tampon, tels que des trous d'ébauche de tampon, dont chacun est approprié pour recevoir et libérer une seule ébauche de tampon pendant un arrêt du dispositif de transfert d'ébauche de tampon et pour retenir l'ébauche de tampon pendant le mouvement du dispositif de transfert d'ébauche de tampon et dans lequel ledit dispositif de transfert de préforme de tampon comprend au moins deux moyens de support de préforme de tampon, tels que des trous de préforme de tampon, dont chacun est approprié pour recevoir et libérer une seule préforme de tampon pendant un arrêt du dispositif de transfert de préforme de tampon et pour retenir la préforme de tampon pendant le mouvement du dispositif de transfert de préforme de tampon.

7. Appareil selon la revendication 6, dans lequel le dispositif de transfert d'ébauche de tampon est configuré pour positionner de manière intermittente et cyclique chacun desdits moyens de support d'ébauche de tampon en face de ladite alimentation de source de sorte qu'une ébauche de tampon peut être insérée longitudinalement dans l'un desdits moyens de support d'ébauche de tampon pendant un arrêt dudit dispositif de transfert d'ébauche de tampon, et/ou dans lequel le dispositif de transfert de préforme de tampon est configuré pour positionner de manière intermittente et cyclique chacun desdits moyens de support de préforme de tampon en face de ladite alimentation de destination de sorte qu'une préforme de tampon peut être libérée longitudinalement de l'un desdits moyens de support de préforme de tampon pendant un arrêt dudit dispositif de transfert de préforme de tampon.

8. Appareil selon l'une quelconque des revendications 5 à 7, dans lequel ledit dispositif de transfert d'ébauche de tampon comprend un axe de pivot pour transférer la première ébauche de tampon de l'alimentation de source à la première presse de tampon par un mouvement de pivotement autour dudit axe et pour transférer ensuite la seconde ébauche de tampon de l'alimentation de source à la seconde presse de tampon par un mouvement de pivotement autour dudit axe de pivot.

9. Appareil selon l'une quelconque des revendications 5 à 8, dans lequel ledit dispositif de transfert de préforme de tampon comprend un axe de pivot pour transférer la première préforme de tampon de la première presse de tampon à l'alimentation de destination par un mouvement de pivotement autour dudit axe et pour transférer ensuite la seconde préforme de tampon de ladite seconde presse de tampon à l'alimentation de destination par un mouvement de pivotement autour dudit axe de pivot.

10. Appareil selon l'une quelconque des revendications 5 à 9, dans lequel lesdites presses de tampon sont des presses de tampon radiales.

11. Appareil selon l'une quelconque des revendications 5 à 10, comprenant un arbre central raccordé de manière fonctionnelle à chacune desdites presses, de préférence via un levier, et l'arbre central raccordé, de manière fonctionnelle, au dispositif de transfert d'ébauche de tampon et/ou au dispositif de transfert de préforme de tampon, de sorte que l'arbre central est configuré pour actionner conjointement les presses de tampon, et le dispositif de transfert d'ébauche de tampon et/ou le dispositif de transfert de préforme de tampon.

12. Dispositif de transfert d'ébauche de tampon pour transférer des ébauches de tampon d'une alimentation de source à un certain nombre d'au moins deux presses de tampon, ledit dispositif comprenant des moyens de support d'ébauche de tampon, tels que des trous d'ébauche de tampon, dont chacun est approprié pour recevoir et libérer une seule ébauche de tampon pendant un arrêt du dispositif de transfert d'ébauche de tampon et pour retenir l'ébauche de tampon pendant le mouvement du dispositif de transfert d'ébauche de tampon,
dans lequel le dispositif de transfert d'ébauche de tampon est configuré pour positionner de manière intermittente et cyclique chacun desdits moyens de support d'ébauche de tampon au niveau de ladite alimentation de source et au moins l'une desdites presse de tampon,
**caractérisé en ce que** :
le dispositif de transfert d'ébauche de tampon comprend un certain nombre de moyens de support d'ébauche de tampon, ledit nombre étant de deux ou plus et ledit nombre étant au moins égal audit nombre de presses de tampon, et
dans lequel le dispositif est configuré, pendant un seul arrêt du dispositif de transfert d'ébauche de tampon, pour :
libérer une première ébauche de tampon d'un premier moyen de support d'ébauche de tampon du dispositif de transfert d'ébauche de tampon dans l'une des presses de tampon et recevoir une seconde ébauche de tampon de l'alimentation de source dans un second moyen de support d'ébauche de tampon du dispositif de transfert d'ébauche de tampon ; ou bien
libérer une première ébauche de tampon d'un premier moyen de support d'ébauche de tampon du dispositif de transfert d'ébauche de tampon dans une première presse de tampon et libérer une seconde ébauche de tampon d'un second moyen de support d'ébauche de tampon du dispositif de transfert d'ébauche de tampon dans une seconde presse de tampon.

13. Dispositif de transfert de préforme de tampon pour transférer des préformes de tampon d'un certain nombre d'au moins deux presses de tampon à une alimentation de destination, ledit dispositif comprenant des moyens de support de préforme de tampon, tels que des trous de préforme de tampon, dont chacun est approprié pour recevoir et libérer une seule préforme de tampon pendant un arrêt du dispositif de transfert de préforme de tampon et pour retenir la préforme de tampon pendant le mouvement du dispositif de transfert de préforme de tampon,
dans lequel le dispositif de transfert de préforme de tampon est configuré pour positionner de manière intermittente et cyclique chacun desdits moyens de support de préforme de tampon à ladite alimentation de destination et au moins l'une desdites presses de tampon,
**caractérisé en ce que** :
le dispositif de transfert de préforme de tampon comprend un certain nombre de moyens de support de préforme de tampon, ledit nombre étant de deux ou plus et ledit nombre étant au moins égal audit nombre de presses de tampon, et
dans lequel le dispositif est configuré, pendant un seul arrêt du dispositif de transfert de préforme de tampon pour :
libérer une première préforme de tampon d'un premier moyen de support de préforme de tampon du dispositif de transfert de préforme de tampon dans l'alimentation de destination et recevoir une seconde préforme de tampon de l'une des presses de tampon dans un second moyen de support de préforme de tampon du dispositif de transfert de préforme de tampon ; ou bien
recevoir une première préforme de tampon d'une première presse de tampon dans un premier moyen de support de préforme de tampon du dispositif de transfert de préforme de tampon et recevoir une seconde préforme de tampon d'une seconde presse de tampon dans un second moyen de support de préforme de tampon du dispositif de transfert de préforme de tampon.

14. Procédé pour transférer des ébauches de tampon d'une alimentation de source à un certain nombre d'au moins deux presses de tampon, comprenant les étapes consistant à :
(i) transférer une première ébauche de tampon d'une alimentation de source à une première presse de tampon en pivotant ladite première ébauche de tampon autour d'un axe de pivot ;
(ii) transférer une seconde ébauche de tampon de ladite alimentation de source à une seconde presse de tampon, **caractérisé en ce que** ledit transfert est réalisé après l'étape (i) en faisant pivoter ladite seconde ébauche de tampon autour dudit axe de pivot ;
(iii) comprimer ladite première ébauche de tampon dans une première préforme de tampon ;
(iv) comprimer ladite seconde ébauche de tampon dans une seconde préforme de tampon ;
(v) libérer ladite première préforme de tampon de ladite première presse et transférer ladite première préforme de tampon de ladite première presse à une alimentation de destination ;
(vi) libérer ladite seconde préforme de ladite seconde presse et transférer ladite seconde préforme de tampon de ladite seconde presse à ladite alimentation de destination.

15. Procédé pour transférer des préformes de tampon d'un certain nombre d'au moins deux presses de tampon à une alimentation de destination, comprenant les étapes consistant à :
(I) transférer une première ébauche de tampon d'une alimentation de source à une première presse de tampon et insérer ladite première ébauche de tampon dans ladite première presse de tampon ;
(II) transférer une seconde ébauche de tampon de ladite alimentation de source à une seconde presse de tampon et insérer ladite seconde ébauche de tampon dans ladite seconde presse de tampon ;
(III) comprimer ladite première ébauche de tampon dans une première préforme de tampon ;
(IV) comprimer ladite seconde ébauche de tampon dans une seconde préforme de tampon ;
(V) transférer ladite première préforme de tampon de ladite première presse de tampon à une alimentation de destination en faisant pivoter ladite première préforme de tampon autour d'un axe de pivot ;
(VI) transférer ladite seconde préforme de tampon de ladite seconde presse de tampon à ladite alimentation de destination, **caractérisé en ce que** ledit transfert est réalisé après l'étape (V) en faisant pivoter ladite seconde préforme de tampon autour dudit axe de pivot.
